# EUROPEAN PATENT APPLICATION

(11) **EP 2 060 553 A1**
(43) Date of publication of application: **20.05.2009**
(21) Application number: 07254454.7
(22) Date of filing: 14.11.2007
(51) Int. Cl.: C07C 29/149, C07C 31/08, C07C 51/12, C07C 53/08, C07C 67/08, C07C 69/14

(54) **Process for the conversion of hydrocarbons into alcohol**

(71) Applicant: BP p.l.c., London SW1Y 4PD (GB)
(72) Inventor: Daniel, Berian, John, Hull East Yorkshire HU12 8DS (GB); Gracey, Benjamin, Patrick, Hull East Yorkshire HU12 8DS (GB)
(74) Representative: De Kezel, Eric

(57) **Abstract**

The present invention relates to a process for the conversion of a carbonaceous feedstock to ethanol; wherein the carbonaceous feedstock is first converted to ethanoic acid, which is then hydrogenated and converted into ethanol.

## Description

The present invention relates to a process for the conversion of ethanoic acid to ethanol.

In particular the present invention relates to a process for the conversion of a carbonaceous feedstock to ethanol; wherein the carbonaceous feedstock is first converted to ethanoic acid, which is then hydrogenated and converted into ethanol.

In recent years increased use and demand for alcohols such as methanol, ethanol and higher alcohols has led to a greater interest in processes relating to alcohol production. The said alcohols may be produced by the fermentation of, for example, sugars and/or cellulosic materials.

Alternatively alcohols, such as ethanol, may be produced from synthesis gas. Synthesis gas refers to a combination of hydrogen and carbon oxides produced in a synthesis gas plant from a carbon source such as natural gas, petroleum liquids, biomass and carbonaceous materials including coal, recycled plastics, municipal wastes, or any organic material. Thus, alcohol and alcohol derivatives may provide non-petroleum based routes for the production of valuable chemicals and fuels.

Generally, the production of alcohols, for example methanol, takes place via three process steps: synthesis gas preparation, methanol synthesis, and methanol purification. In the synthesis gas preparation step, an additional stage maybe employed whereby the feedstock is treated, e.g. the feedstock is purified to remove sulphur and other potential catalyst poisons prior to being converted into synthesis gas. This treatment can also be conducted after syngas preparation; for example, when coal or biomass is employed.

The reaction to produce alcohol(s) from syngas is generally exothermic. The formation of C2 and C2+ alcohols is believed to proceed via the formation of methanol for modified methanol catalysts and cobalt molybdenum sulphide catalysts. However, the production of methanol is equilibrium limited and thus requires high pressures in order to achieve viable yields. Hence, pressure can be used to increase the yield, as the reaction which produces methanol exhibits a decrease in volume, as disclosed in U.S. Pat. No. 3,326,956. Improved catalysts have now allowed viable rates of methanol formation to be achieved at reduced reaction temperatures, and
hence allow commercial operation at lower reaction pressures, e.g. a copper oxide-zinc oxide-alumina catalyst that typically operates at a nominal pressure of 5-10 MPa and temperatures ranging from approximately 150 DEG C. to 450 DEG C. over a variety of catalysts, including CuO/ZnO/Al2 03, CuO/ZnO/Cr2 03, ZnO/Cr2O3, and supported Fe, Co, Ni, Ru, Os, Pt, and Pd catalysts. A low-pressure, copper-based methanol synthesis catalyst is commercially available from suppliers such as BASF, ICI Ltd. of the United Kingdom, and Haldor-Topsoe. Methanol yields from copper-based catalysts are generally over 99.5% of the converted CO+CO2 present. Water is a by-product of the conversion of CO2 to methanol and the conversion of CO synthesis gas to C2 and C2+ oxygenates. In the presence of an active water gas-shift catalyst, such as a methanol catalyst or a cobalt molybdenum catalyst the water equilibrates with the carbon monoxide to give CO2 and hydrogen. A paper entitled, "Selection of Technology for Large Methanol Plants," by Helge Holm-Larsen, presented at the 1994 World Methanol Conference, Nov. 30-Dec. 1, 1994, in Geneva, Switzerland, reviews the developments in methanol production and shows how further reduction in costs of methanol production will result in the construction of very large plants with capacities approaching 10,000 metric tonnes per day.

Other processes, for the production of C2 and C2+ alcohol(s), include the processes described hereinafter; US 4,122,110 relates to a process for manufacturing alcohols, particularly linear saturated primary alcohols, by reacting carbon monoxide with hydrogen at a pressure between 20 and 250 bars and a temperature between 150 and 400 DEG C., in the presence of a catalyst, characterized in that the catalyst contains at least 4 essential elements: (a) copper (b) cobalt (c) at least one element M selected from chromium, iron, vanadium and manganese, and (d) at least one alkali metal.

US 4,831,060 relates to the production of mixed alcohols from carbon monoxide and hydrogen gases using a catalyst, with optionally a co-catalyst, wherein the catalyst metals are molybdenum, tungsten or rhenium, and the co-catalyst metals are cobalt, nickel or iron. The catalyst is promoted with a Fischer-Tropsch promoter like an alkali or alkaline earth series metal or a smaller amount of thorium and is further treated by sulfiding. The composition of the mixed alcohols fraction can be selected by selecting the extent of intimate contact among the catalytic components.

Journal of Catalysis 114, 90-99 (1988) discloses a mechanism of ethanol formation from synthesis gas over CuO/ZnO/Al2O3. The formation of ethanol from carbon monoxide and hydrogen over a CuO/ZnO methanol catalyst is studied in a fixed-bed microreactor by measuring the isotopic distribution of the carbon in the product ethanol when isotopically-enriched C¹³ methanol was added to the feed.

As the importance of ethanol is ever increasing in today's world, so is the need and desire to produce ethanol with a higher carbon efficiency, a higher conversion and an improved selectivity from a carbonaceous feedstock. Hence, the present invention provides a process that allows one to produce ethanol from a carbonaceous feedstock, with improved carbon efficiency, a higher selectivity and, in particular, with a more efficient conversion to ethanol, including a more efficient process step for the conversion of ethanoic acid to ethanol.

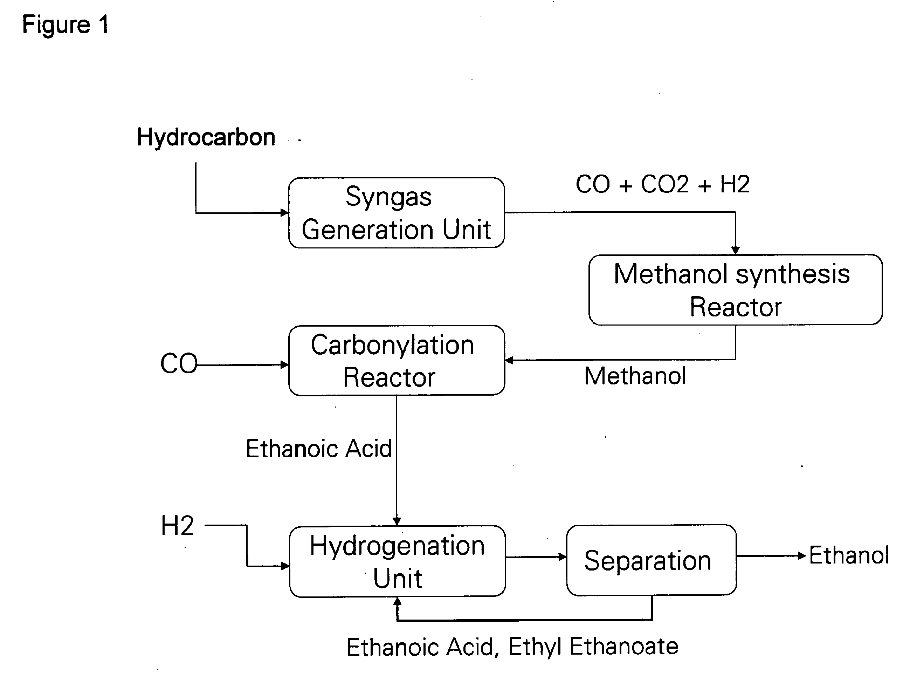

Figure 1 represents an embodiment of a process scheme according to the present invention wherein the references correspond to those used in the present description and appending claims.

Thus, the present invention relates to a process for the conversion of ethanoic acid into ethanol characterised by the following steps:
1. introducing ethanoic acid and hydrogen into a hydrogenation unit to produce a stream comprising ethanol, ethyl ethanoate and unreacted ethanoic acid,
2. separating the ethanol produced in step 1, from the ethyl ethanoate and unreacted ethanoic acid,
3. recycling the separated ethyl ethanoate and unreacted ethanoic acid, from step 2, into the hydrogenation unit of step 1, and
4. recovering the ethanol from step 2.

The present invention also relates to a process for the conversion of methanol into ethanol, characterised by the following steps:
1. introducing methanol, together with carbon monoxide, into a carbonylation reactor to produce ethanoic acid,
2. introducing ethanoic acid, from step 1 and hydrogen into a hydrogenation unit to produce a stream comprising ethanol, ethyl ethanoate and unreacted ethanoic acid,
3. separating the ethanol produced in step 2, from the ethyl ethanoate and unreacted ethanoic acid,
4. recycling the separated ethyl ethanoate and unreacted ethanoic acid, from step 3, into the hydrogenation unit of step 2, and
5. recovering the ethanol from step 3.

Furthermore, the present invention relates to a process for the conversion of a hydrocarbon feedstock into ethanol, whereby the hydrocarbon feedstock is first converted into synthesis gas, which is subsequently converted into ethanol, characterised by the following consecutive steps:
1. introducing a hydrocarbon feedstock into a synthesis gas generation unit to produce synthesis gas,
2. introducing the synthesis gas, produced in step 1, into a methanol synthesis reactor to produce methanol,
3. introducing methanol, from step 2, together with carbon monoxide, into a carbonylation reactor to produce ethanoic acid,
4. introducing ethanoic acid, from step 3 and hydrogen into a hydrogenation unit to produce a stream comprising ethanol, ethyl ethanoate and unreacted ethanoic acid,
5. separating the ethanol produced in step 4, from the ethyl ethanoate and unreacted ethanoic acid,
6. recycling the separated ethyl ethanoate and unreacted ethanoic acid, from step 3, into the hydrogenation unit of step 4, and
7. recovering the ethanol from step 5.

According to one aspect of the present invention, the synthesis gas feedstock, a mixture of carbon oxide(s) and hydrogen, that is used to produce the methanol feed stream, is preferably produced from a hydrocarbon feedstock.

The hydrocarbon feedstock is preferably a carbonaceous material, for example biomass, plastic, naphtha, refinery bottoms, crude syngas (from underground coal gasification or biomass gasification), smelter off gas, municipal waste, coal bed methane, coal, and/or natural gas, with coal and natural gas being the preferred sources, and natural gas being the most preferable source. To one skilled in art a combination of sources can also be used, for example coal and natural gas to advantageously increase the hydrogen to carbon ratio.

Natural gas commonly contains a range of hydrocarbons (e.g. C1-C3 alkanes), in which methane predominates. In addition to this, natural gas will usually contain nitrogen, carbon dioxide and sulphur compounds. Preferably the nitrogen content of the feedstock is less than 40 wt %, more preferably less than 10 wt % and most preferably less than 1 wt %.

Processes for producing mixtures of carbon oxide(s) and hydrogen (commonly known as synthesis gas), in a syngas reactor, are well known. Each method has its advantages and disadvantages, and the choice of using a particular reforming process over another is governed by economic and available feed stream considerations, as well as by the desire to obtain the optimum (H2-CO2):(CO+CO2) molar ratio in the resulting synthesis gas that is suitable for further chemical processing. A discussion of the available synthesis gas production technologies is provided in both "Hydrocarbon Processing" V78, N.4, 87-90, 92-93 (April 1999) and "Petrole et Techniques", N. 415, 86-93 (July-August 1998), and are both hereby incorporated by reference.

It is also known that the synthesis gas may be obtained by catalytic partial oxidation of hydrocarbons in a microstructured reactor as exemplified in "IMRET 3: Proceedings of the Third International Conference on Microreaction Technology", Editor W Ehrfeld, Springer Verlag, 1999, pages 187-196. Alternatively, the synthesis gas may be obtained by short contact time catalytic partial oxidation of hydrocarbonaceous feedstocks as described in EP 0303438. The synthesis gas can also be obtained via a "Compact Reformer" process as described in "Hydrocarbon Engineering", 2000, 5, (5), 67-69; "Hydrocarbon Processing", 79/9, 34 (September 2000); "Today's Refinery", 15/8, 9 (August 2000); WO 99/02254; and WO 200023689.

Typically, for commercial syngas production the pressure at which the synthesis gas is produced from a steam reformer ranges from approximately 0.1 to 10 MPa, preferably 2 to 3 MPa and the temperatures at which the synthesis gas exits the reformer ranges from approximately 700 to 1000 DEG C. Likewise, for commercial syngas production the pressure at which the synthesis gas is produced from an auto-thermal reformer ranges from approximately 1 to 100 bar, preferably 20 to 50 bar and the temperatures at which the synthesis gas exits the reformer ranges from approximately 700 to 1300 DEG C. Where the high temperatures are necessary in order to produce a favourable equilibrium for syngas production, and to avoid metallurgy problems associated with carbon dusting. The synthesis gas contains a molar ratio of (H2-CO2):(CO+CO2) ranging from 0.8 to 3.0, which is dependent on the hydrocarbon feedstock(s) and the method of reforming used. For example, when natural gas is used as the hydrocarbon feedstock for steam reforming, the syngas obtained usually has a maximum (H2-CO2):(CO+CO2) ratio of 3.0. However, when natural gas is used as the hydrocarbon feedstock for auto-thermal reforming, the syngas obtained usually has a (H2-CO2):(CO+CO2) ratio of 1.5.

According to a preferred embodiment of the present invention, the molar ratio, (H2-CO2):(CO+CO2), of the syngas stream exiting the syngas generation unit(s) is greater than 1.6, more preferably greater than 1.8 and most preferably greater than 2.0. Preferably, the molar ratio, (H2-CO2):(CO+CO2), of said syngas stream exiting the syngas generation unit(s) is less than 3.0, preferably less than 2.75, more preferably less than 2.4 and most preferably less than 2.2.

According to another embodiment of this invention when the hydrocarbon feed stock used for syngas generation is not a aliphatic hydrocarbon (e.g. coal , aromatic material, biomass) the molar ratio (H2-CO2):(CO+CO2) of the exit syngas is preferably adjusted to the target value by addition of hydrogen or removal of CO2.

According to a preferred embodiment of the present invention, the exit stream obtained from the syngas reactor (e.g. using a steam reformer), comprises essentially a mixture of carbon oxide(s) and hydrogen. It can also comprise water, nitrogen and traces of unconverted hydrocarbons (e.g. C1-C3 alkanes).

According to a preferred embodiment of the present invention, during synthesis gas generation, an additional stage may be employed whereby the feedstock is first purified to remove sulphur and other potential catalyst poisons (such as halides or metals e.g. Hg) prior to being converted into synthesis gas; alternatively this treatment can also be performed after syngas preparation for example, when coal or biomass are used.

According to an embodiment of the present invention, at least part of the said syngas stream is then introduced into a methanol synthesis unit, in order to produce a stream comprising methanol. Preferably the molar ratio, (H2-CO2):(CO+CO2), of said syngas feed stream fed into the methanol synthesis reactor is greater than 1.6, more preferably greater than 1.8 and most preferably greater than 2.0. Preferably the molar ratio, (H2-CO2):(CO+CO2), of said syngas feed stream fed into the methanol synthesis reactor is less than 3.0, more preferably less than 2.5 and most preferably less than 2.2.

According to a preferred embodiment of the present invention, the methanol synthesis unit may be any reactor that is suitable for producing methanol, for example a fixed bed reactor, which can be run in adiabatic or isothermal mode e.g. a multi-tubular reactor; or a fluidised bed reactor.

Preferably the methanol synthesis unit is operated at a temperature of more than 200°C, preferably more than 220°C and most preferably more than 240°C; and less than 310°C, preferably less than 300°C and most preferably less than 290°C. Preferably the methanol synthesis unit is operated at pressure of more than 2 MPa and preferably more than 5 MPa; and less than 10 MPa and preferably less than 9MPa. In fact, since methanol synthesis is an exothermic reaction, the chosen temperature of operation is governed by a balance of promoting the forward reaction (i.e. by not adversely affecting the equilibrium) and aiding the rate of conversion (i.e. higher productivity).

The catalysts used for methanol synthesis can be divided into 2 groups:
i. the high pressure zinc catalysts, composed of zinc oxide and a promoter; and
ii. low pressure copper catalysts, composed of zinc oxide, copper oxide, and a promoter.

Hence, according to a preferred embodiment of the present invention, the preferred methanol synthesis catalyst is a mixture of copper, zinc oxide, and a promoter such as, chromia or alumina. Under the aforementioned operating conditions, these said mixtures can catalyse the production of methanol from carbon monoxide and hydrogen with a high selectivity.

Additionally by-products such as methane, ethanol and other higher alcohols may also be produced during methanol synthesis. According to a preferred embodiment of this aspect of the present invention, the stream exiting the methanol synthesis reactor is subsequently purified to remove said by-products by any methods known to those in the art.

According to another aspect of the present invention, a methanol stream, together with a substantially pure carbon monoxide stream, are introduced into a carbonylation reactor. Preferably, at least part of the said methanol stream emanates from the aforementioned methanol synthesis unit, however said methanol stream may also emanate from another suitable source, such as a bio-fermentation process and/or pyrolysis (e.g. wood pyrolysis).

Preferably at least a part of the said carbon monoxide stream is obtained from the aforementioned syngas generation stage. This is preferably performed by first removing carbon dioxide and water from the generated synthesis gas followed by a cryogenic separation to isolate the substantially pure carbon monoxide from the hydrogen. Alternative methods of separation, such as membrane separation technologies can also be employed. Alternatively, said carbon monoxide stream may also be obtained from another suitable source, such as another chemical process (e.g. off-gas from steel manufacture). Said carbon monoxide stream(s) may still contain inert impurities such as carbon dioxide, methane, nitrogen, noble gases, water and C1 to C4 paraffinic hydrocarbons, which are preferably removed before use.

According to this aspect of the present invention, the step of introducing methanol, together with carbon monoxide, into a carbonylation reactor is performed under conditions favourable for producing ethanoic acid.

There are many examples in the prior art which disclose carbonylation processes that can be suitably used in the present invention.

For example, such carbonylation processes can be made in the presence of iridium catalysts as described in US 3772380. UK patent GB 1276326 also describes the preparation of mono-carboxylic acids by carbonylation of alcohols in the presence of rhodium or iridium catalysts, halogen promoters and water or an alcohol, ether or ester.

Carbonylation processes in the presence of ruthenium and osmium catalysts can also be suitably used in the present invention. Thus, UK patents GB 1234641 and GB 1234642 describe a process for the production of an organic acid by carbonylation of an alcohol in the presence of a noble metal catalyst selected from iridium, platinum, palladium, osmium and ruthenium and their compounds and a promoter which is halogen or halogen compound. According to Jenner et al in J.Mol. Catalysis 40 (1987) 71-82 ruthenium compounds are effective carbonylation catalysts for converting primary alcohols into acids at high CO pressures. Standard conditions of 45 MPa CO pressure were used in the reported experiments. For example, UK patent application GB 2029409 describes a process for the preparation of aliphatic carboxylic acids by reacting carbon monoxide with alcohols at an elevated pressure of 3.4 MPa or greater in the presence of a ruthenium catalyst and halogen-containing promoter.

According to a preferred embodiment of this aspect of the present invention, the carbonylation process takes place in the presence of an iridium catalyst together with at least one promoter; indeed, such catalyst systems have proven to have beneficial effects on the rate of carbonylation of methanol. Said carbonylation process is thus preferably performed in the presence of at least a finite concentration of water with a catalyst system comprising:
(a) an iridium catalyst, (b) methyl iodide and (c) at least one promoter.

Thus, according to a preferred embodiment of this aspect of the present invention the process for the production of ethanoic acid by carbonylation of methanol comprises contacting methanol with carbon monoxide, in the liquid reaction composition, in a carbonylation reactor wherein, the liquid reaction composition comprises:
(a) ethanoic acid, (b) an iridium catalyst, (c) methyl iodide, (d) water and (e) at least one promoter.

According to an embodiment of this aspect of the present invention, during the carbonylation process, water may be formed in situ in the liquid reaction composition. For example, water may be produced via by-product formation, generated during methane production. Water may also be generated during the esterification reaction between methanol reactant and ethanoic acid product. Water may also be introduced to the carbonylation reactor together with, or separately from, other components of the liquid reaction composition. Water may be separated from other components of reaction composition withdrawn from the reactor and may be recycled in controlled amounts to maintain a preferred concentration of water in the liquid reaction composition. Preferably, the concentration of water in the liquid reaction composition of the carbonylation reactor is in the range 0.1 to 15% by weight, more preferably 1 to 10% by weight, most preferably 1 to 6.5% by weight.

The iridium catalyst in the liquid reaction composition may comprise any iridium containing compound which is soluble in the liquid reaction composition. The iridium catalyst may be added to the liquid reaction composition for the carbonylation reaction in any suitable form which dissolves in the liquid reaction composition or is convertible to a soluble form. Examples of suitable iridium-containing compounds which may be added to the liquid reaction composition include IrCl3, IrI3, IrBr3, [Ir(CO)2I]2, [Ir(CO)2Cl]2, [Ir(CO)2Br]2, [Ir(CO)2I2]⁻ H⁺, [Ir(CO)2Br2]⁻ H⁺, [Ir(CO)2I4]⁻ H⁺, [Ir(CH3)I3(CO)2]⁻ H⁺, Ir4(CO)12, IrC13.3H2O, IrBr3.3H2O, Ir4(CO)12, iridium metal, Ir2O3, IrO2, Ir(acac)(CO)2, Ir(acac)3, iridium acetate, [Ir3O(OAc)6(H2O)3][OAc] and hexachloroiridic acid [H2IrCl6], preferably, chloride-free complexes of iridium such as acetates, oxalates and acetoacetates which are soluble in one or more of the carbonylation reaction components such as water, alcohol and/or carboxylic acid. Particularly preferred is green iridium acetate which may be used in an acetic acid or aqueous acetic acid solution.

Preferably, the iridium carbonylation catalyst concentration in the liquid reaction composition is in the range 100 to 6000 ppm by weight of iridium, more preferably 700 to 3000 ppm by weight of iridium.

In the process of the present invention at least one promoter is present in the reaction composition. Suitable promoters are preferably selected from the group consisting of ruthenium, osmium, rhenium, cadmium, mercury, zinc, gallium, indium and tungsten, and are more preferably selected from ruthenium and osmium and most preferably is ruthenium. Preferably, the promoter is present in an effective amount up to the limit of its solubility in the liquid reaction composition and/or any liquid process streams recycled to the carbonylation reactor from the acetic acid recovery stage. The promoter is suitably present in the liquid reaction composition at a molar ratio of promoter : iridium of [0.5 to 15] : 1.As noted above, the beneficial effect of a promoter such as ruthenium has been found to be greatest at the water concentration which gives the maximum carbonylation rate at any defined methyl acetate and methyl iodide concentration. A suitable promoter concentration is 400 to 5000 ppm.

The promoter may comprise any suitable promoter metal-containing compound which is soluble in the liquid reaction composition. The promoter may be added to the liquid reaction composition for the carbonylation reaction in any suitable form which dissolves in the liquid reaction composition or is convertible to soluble form.

Examples of suitable ruthenium-containing compounds which may be used as sources of promoter include ruthenium (III) chloride, ruthenium (III) chloride trihydrate, ruthenium (IV) chloride, ruthenium (III) bromide, ruthenium metal, ruthenium oxides, ruthenium (III) formate, [Ru(CO)3I3]⁻ H⁺, [Ru(CO)2I2]n, [Ru(CO)4I2], [Ru(CO)3I2]2, tetra(aceto)chlororuthenium(II,III), ruthenium (III) acetate, ruthenium (III) propionate, ruthenium (III) butyrate, ruthenium pentacarbonyl, trirutheniumdodecacarbonyl and mixed ruthenium halocarbonyls such as dichlorotricarbonylruthenium (II) dimer, dibromotricarbonylruthenium (II) dimer, and other organoruthenium complexes such as tetrachlorobis(4-cymene)diruthenium(II), tetrachlorobis(benzene)diruthenium(II), dichloro(cycloocta-1,5-diene)ruthenium (II) polymer and tris(acetylacetonate)ruthenium (III).

Examples of suitable osmium-containing compounds which may be used as sources of promoter include osmium (III) chloride hydrate and anhydrous, osmium metal, osmium tetraoxide, triosmiumdodecacarbonyl, [Os(CO)4I2], [Os(CO)3I2]2, [Os(CO)3I3]⁻ H⁺, pentachloro- mu -nitrodiosmium and mixed osmium halocarbonyls such as tricarbonyldichloroosmium (II) dimer and other organoosmium complexes.

Examples of suitable rhenium-containing compounds which may be used as sources of promoter include Re2(CO)10, Re(CO)5Cl, Re(CO)5Br, Re(CO)5I, ReCl3.xH2O, [Re(CO)4I]2, [Re(CO)4I2]⁻ H⁺, and ReCl5.yH2O.

Examples of suitable cadmium-containing compounds which may be used include Cd(OAc)2, CdI2, CdBr2, CdCl2, Cd(OH)2, and cadmium acetylacetonate.

Examples of suitable mercury-containing compounds which may be used as sources of promoter include Hg(OAc)2, HgI2, HgBr2, HgCl2, Hg2I2, and Hg2Cl2.

Examples of suitable zinc-containing compounds which may be used as sources of promoter include Zn(OAc)2, Zn(OH)2, ZnI2, ZnBr2, ZnCl2, and zinc acetylacetonate.

Examples of suitable gallium-containing compounds which may be used as sources of promoter include gallium acetylacetonate, gallium acetate, GaCl3, GaBr3, GaI3, Ga2C14 and Ga(OH)3.

Examples of suitable indium-containing compounds which may be used as sources of promoter include indium acetylacetonate, indium acetate, InCl3, InBr3, InI3, InI and In(OH)3.

Examples of suitable tungsten-containing compounds which may be used as sources of promoter include W(CO)6, WC14, WCl6, WBr5, WI2, or C9H12 W(CO)3 and any tungsten chloro-,bromo- or iodo-carbonyl compound.

Preferably, the iridium- and promoter-containing compounds are free of impurities which provide or generate in situ ionic iodides which may inhibit the reaction, for example, alkali or alkaline earth metal or other metal salts.

Ionic contaminants such as, for example, (a) corrosion metals, particularly nickel, iron and chromium and (b) phosphines or nitrogen containing compounds or ligands which may quaternise in situ; should be kept to a minimum in the liquid reaction composition as these will have an adverse effect on the reaction by generating I⁻ in the liquid reaction composition which has an adverse effect on the reaction rate. Some corrosion metal contaminants such as for example molybdenum have been found to be less susceptible to the generation of I⁻. Corrosion metals which have an adverse affect on the reaction rate may be minimised by using suitable corrosion resistant materials of construction. Similarly, contaminants such as alkali metal iodides, for example lithium iodide, should be kept to a minimum. Corrosion metal and other ionic impurities may be reduced by the use of a suitable ion exchange resin bed to treat the reaction composition, or preferably a catalyst recycle stream. Such a process is described in US 4007130. Preferably, ionic contaminants are kept below a concentration at which they would generate 500 ppm [I]⁻, preferably less than 250 ppm [I]⁻ in the liquid reaction composition.

Preferably, the concentration of methyl iodide in the liquid reaction composition is in the range 1 to 20% by weight, preferably 5 to 16% by weight.

The partial pressure of carbon monoxide in the carbonylation reactor is suitably in the range 0.1 to 7 MPa preferably 0.1 to 3.5 MPa and most preferably 0.1 to 1.5 MPa.

The catalyst system used in the carbonylation process of the present invention has been found to be particularly beneficial at relatively low partial pressures of carbon monoxide where the rate of reaction may be dependent upon the carbon monoxide partial pressure. Under these conditions, it has been found that the catalyst system has the advantage of providing an increased rate of reaction over catalyst systems without the promoters of the present invention. This advantage allows for an increased rate of reaction under conditions when the carbon monoxide partial pressure is relatively low, for example due to a low total pressure in the carbonylation reactor or due to high vapour pressure of components of the liquid reaction composition, e.g. at high methyl ethanoate concentration in the liquid reaction composition or due to a high concentration of inert gases (for example nitrogen and carbon dioxide) in the carbonylation reactor. The catalyst system may also have advantages of increasing rate of carbonylation when the rate of reaction is reduced by the availability of carbon monoxide in solution in the liquid reaction composition resulting from mass transfer limitations, for example due to poor agitation.

The pressure of the carbonylation reaction is suitably in the range 0.9 to 19.9 MPa, preferably 0.9 to 9.9 MPa, most preferably 1.4 to 4.9 MPa. The temperature of the carbonylation reaction is suitably in the range 100 to 300 DEG C, preferably in the range 150 to 220 DEG C.

Ethanoic acid may advantageously be used as a solvent for said carbonylation reaction.

Corrosion metals, particularly nickel, iron and chromium should preferably be kept to a minimum in the liquid reaction composition as these may have an adverse effect in said carbonylation reaction.

The carbonylation process of the present invention may be performed as a batch or continuous process, preferably as a continuous process and may be performed in any suitable reactor, known to those skilled in the art.

The ethanoic acid product may be removed from the reactor by withdrawing liquid reaction composition and separating the ethanoic acid product by one or more flash and/or fractional distillation stages from the other components of the liquid reaction composition such as iridium catalyst, ruthenium and/or osmium and/or indium promoter, methyl iodide, water and unconsumed reactants which may be recycled to the reactor to maintain their concentrations in the liquid reaction composition. The ethanoic acid product may also be removed as a vapour from the stream exiting the carbonylation reactor.

According to the present invention, ethanoic acid is introduced into a hydrogenation unit together with hydrogen to produce a stream comprising ethyl ethanoate and ethanol. In addition to the production of ethanol and ethyl ethanoate, the hydrogenation process also produces water, other reaction products (e.g. trace amounts of methane, ethane, diethyl ether and ethanal) and unreacted starting materials (e.g. unreacted ethanoic acid and unreacted hydrogen).

As mentioned above the hydrogenation process also produces ethyl ethanoate and water. The proportion of ethyl ethanoate present in the exit stream will be determined by the nature of the catalyst and the degree of conversion. The proportion of ethyl ethanoate may be further increased, if desired, by introducing an acidic function into the catalyst to promote in situ esterification. It is also possible to reduce the proportion of ethyl ethanoate by use of the same acidic function by co-feeding water.

It is has also been found to be an advantage of this process, that the selectivity of the ethanoic acid hydrogenation to ethanol can be further increased at the expense of undesirable by-products, such as the aforementioned alkanes (e.g. ethane and methane).

According to a preferred embodiment of the present invention, at least a part, preferably all, of the said ethanoic acid stream emanates from the aforementioned carbonylation reactor; however in practice the said ethanoic acid stream may also originate from another suitable source, such as wood pyrolysis and/or as a by-product of a fermentation process to produce alcohol(s).

Preferably at least a part of the said hydrogen stream emanates from the syngas generation procedure (i.e. it is obtained during the aforementioned carbon monoxide/hydrogen separation), where, if need be, the hydrogen content can be further increased by subjecting the said syngas to a water gas shift reaction and a subsequent hydrogen separation.

Alternatively the hydrogen stream may originate from a variety of other chemical processes, including ethene crackers, styrene manufacture and catalytic reforming. However, it is known that the main commercial processes for purposeful generation of hydrogen are autothermal reforming, steam reforming and partial oxidation of hydrocarbon feedstocks such as natural gas, coal, coke, deasphalter bottoms, refinery residues and biomass. Hydrogen may also be produced by electrolysis of water.

The overall choice of technology for producing hydrogen is generally determined by the following economic considerations and factors:
i. feedstock cost
ii. feedstock availability
iii. capital cost
iv. local energy and operating costs; and
v. environmental considerations

The catalyst(s) employed in the hydrogenation unit, may be selected from a precious metal based catalyst, comprising of at least one noble metal from Group VIII of the periodic table (CAS version, e.g. Fe, Ru, Os, Co, Rh, Ir, Ni, Pd, Pt) and at least one of the metals chosen from rhenium, tungsten and/or molybdenum; and optionally an additional metal, that is capable of alloying with said Group VIII noble metal.

According to a preferred embodiment of the present invention, the catalyst(s) employed in the hydrogenation unit is a palladium based catalyst. Wherein the preferred palladium based catalyst is a supported catalyst which comprises palladium and preferably rhenium and/or silver.

All of the aforementioned catalysts may be supported on any suitable support known to those skilled in the art; non-limiting examples of such supports include carbon, silica, titania, clays, aluminas and mixed oxides, carbon being the preferred support.

According to a preferred embodiment of the present invention, the catalyst(s) employed is heterogeneous.

The hydrogenation process may be operated in a gas phase, or a mixed gas/liquid phase regime. Preferably, the process is operated in a mixed gas/liquid phase; the mixed gas/liquid phase is where a proportion of the substrate (consisting of ethanoic acid and ethyl ethanoate) to be hydrogenated is in the liquid phase. The hydrogenation can be conducted in batch or semi continuous or continuous mode. Continuous mode of operation is the most preferred.

The hydrogenation reaction can be conducted in adiabatic or isothermal mode; where adiabatic mode of operation is preferred. Suitable reactors include single, or a plurality, of adiabatic bed reactors which can be used in series or parallel. For reactors utilised in series, heat exchangers and/or intercoolers and/or additional reactant and/or recycle of intermediates can be employed in between successive reactors to control the reaction temperature. The preferred adiabatic temperature rise is less than 50°C, preferably less than 25°C and most preferably less than 10°C. The preferred use of adiabatic reactors is in series. The adiabatic reactors may be operated at different temperatures depending on composition of the individual reactor feeds.

The applicants have unexpectedly found that the use of a mixed gas/liquid feed to the hydrogenation unit, under adiabatic mode of operation, affords a higher selectivity towards the production of ethanol as well as providing a method for reducing the exotherm of the reaction, thus increasing the lifetime of the hydrogenation catalyst.

The hydrogenation can also be conducted in multi-tubular reactors in which case a cooling/heating medium is circulated around the tubes to control the temperature. For exothermic reactions, as such, there will be a radial temperature gradient in the reactor; the preferred gradient is less than 50°C preferably less than 25°C most preferably less than 10°C. The preferred flow regime in this type of reactor is turbulent rather than laminar, this corresponds to a Reynolds number greater than 2100 (where the velocity is approximated by velocity in an unpacked tube).

The hydrogenation reaction can also be conducted in other reactor types such as fluidised bed, spinning basket, buss loop, and heat exchanger reactors. A mixed liquid/gas phase hydrogenation reaction can be conducted with co-flow or counterflow of the hydrogen and vapour to the liquid (e.g. a bubble reactor). The preferred mode of operation of gas/liquid reactors is co-flow, also known as trickle bed operation; this can be conducted in at least one tubular and/or multi-tubular reactor in series. The hydrogenation reaction may change from a mixed gas/liquid phase to a fully vapour phase reaction, as the reaction proceeds down the reactor. The mixed phase hydrogenation can also be conducted in other types of reactors, or within a combination of different reactors, for example in a slurry or stirred tank reactor with, or without, external circulation and optionally operated as a cascade or stirred tanks, a loop reactor or a Sulzer mixer-reactor.

The hydrogenation reactor(s) preferably operate at a temperature of more than 150 °C, but less than 290 °C.

According to a preferred embodiment of the present invention the reaction temperature is more than 180°C, preferably more than 200°C and most preferably more than 220°C; and less than 290°C, preferably less than 270°C and most preferably less than 250°C.

The hydrogenation reaction may be operated at a pressure of more than 3 MPa, preferably at a pressure of more than 5 MPa and most preferably at a pressure of more than 7 MPa; and at a pressure of less than 15 MPa, more preferably at a pressure less than 13 MPa and most preferably at a pressure less than 9 MPa.

According to a preferred embodiment of the present invention, the hydrogenation unit(s) are operated such that, the feed mixture introduced into the reactor(s) is preferably below the dew point temperature of the feed mixture.

For the purposes of the present invention and appending claims, the 'dew point temperature' is defined as being a threshold temperature. For example, for a given mixture, at a given pressure, if the system temperature is raised to above the dew point temperature, the mixture will exist as a dry gas. Likewise below the dew point temperature, the mixture will exist as a vapour containing some liquid.

The Gas Hourly Space Velocity (GHSV- which is defined as the rate of gas volume per catalyst volume per hour at standard temperature and pressure), which is defined as the rate of gas volume per catalyst volume per hour, for continuous operation may be in the range 50 to 50,000 h⁻¹, preferably from 2,000 to 30,000 h⁻¹ and most preferably from 3,000 to 6,000 h⁻¹.

The liquid substrate introduced into the hydrogenation unit preferably has a Liquid Hourly Space Velocity (LHSV- which is defined as the rate of liquid volume per catalyst volume per hour at standard temperature and pressure), which is defined as the rate of liquid volume per catalyst volume per hour, may be less than 10hr⁻¹, more preferably less than 5hr⁻¹ and most preferably less than 3hr⁻¹; for example, a typical LHSV for normal operation is approximately 1hr⁻¹.

According to the present invention, the stream exiting the hydrogenation unit is then subjected to a separation stage (e.g. distillation), whereby a fraction comprising ethanol is separated and recovered; and an unreacted fraction comprising ethanoic acid, ethyl ethanoate and hydrogen is also separated and recovered and recycled back into said hydrogenation unit.

By performing the aforementioned recycle of the unreacted components (e.g. ethanoic acid, ethyl ethanoate and hydrogen) the applicants were able to achieve an even higher conversion towards ethanol, whilst retaining high selectivity towards ethanol.

The separation stage may be performed by any means known to those skilled in the art that is suitable for separating the said stream, e.g. a sieve tray column, a packed column, a bubble cap column or a combination thereof.

According to a preferred embodiment of the present invention, the molar ratio of hydrogen to [ethanoic acid and ethyl ethanoate] that is introduced into the hydrogenation unit, is greater than 2:1, preferably the molar ratio is greater than 4:1 and most preferably the molar ratio is greater than 5:1; and is less than 100:1, preferably less than 50:1 and most preferably less than 15:1.

It should be noted that whilst all of the aforementioned temperature and pressure operating conditions form preferred embodiments of the present invention, they are not, by any means, intended to be limiting, and the present invention hereby includes any other pressure and temperature operating conditions that achieve the same effect.

## Claims

1. Process for the conversion of ethanoic acid into ethanol **characterised by** the following steps:
1) introducing ethanoic acid and hydrogen into a hydrogenation unit to produce a stream comprising ethanol, ethyl ethanoate and unreacted ethanoic acid,
2) separating the ethanol produced in step 1, from the ethyl ethanoate and unreacted ethanoic acid,
3) recycling the separated ethyl ethanoate and unreacted ethanoic acid, from step 2, into the hydrogenation unit of step 1, and
4) recovering the ethanol from step 2.

2. Process for the conversion of methanol into ethanol, **characterised by** the following steps:
1) introducing methanol, together with carbon monoxide, into a carbonylation reactor to produce ethanoic acid,
2) introducing ethanoic acid, from step 1 and hydrogen into a hydrogenation unit to produce a stream comprising ethanol, ethyl ethanoate and unreacted ethanoic acid,
3) separating the ethanol produced in step 2, from the ethyl ethanoate and unreacted ethanoic acid,
4) recycling the separated ethyl ethanoate and unreacted ethanoic acid, from step 3, into the hydrogenation unit of step 2, and
5) recovering the ethanol from step 3.

3. Process for the conversion of a hydrocarbon feedstock into ethanol, whereby the hydrocarbon feedstock is first converted into synthesis gas, which is subsequently converted into ethanol, **characterised by** the following consecutive steps:
1) introducing a hydrocarbon feedstock into a synthesis gas generation unit to produce synthesis gas,
2) introducing the synthesis gas, produced in step 1, into a methanol synthesis reactor to produce methanol,
3) introducing methanol, from step 2, together with carbon monoxide, into a carbonylation reactor to produce ethanoic acid,
4) introducing ethanoic acid, from step 3 and hydrogen into a hydrogenation unit to produce a stream comprising ethanol, ethyl ethanoate and unreacted ethanoic acid,
5) separating the ethanol produced in step 4, from the ethyl ethanoate and unreacted ethanoic acid,
6) recycling the separated ethyl ethanoate and unreacted ethanoic acid, from step 3, into the hydrogenation unit of step 4, and
7) recovering the ethanol from step 5.

4. Process according to any of claims 2 or 3 wherein the step of producing ethanoic acid by carbonylation of methanol comprises contacting methanol with carbon monoxide, in the liquid reaction composition, in a carbonylation reactor wherein, the liquid reaction composition comprises: (a) ethanoic acid, (b) an iridium catalyst, (c) methyl iodide, (d) water and (e) as a promoter, at least one of ruthenium, osmium and indium.

5. Process according to claim 4 wherein the concentration of water in the liquid reaction composition of the carbonylation reactor is in the range 0.1 to 15% by weight, more preferably 1 to 15% by weight, most preferably 1 to 10% by weight.

6. Process according to any of the preceding claims wherein the catalyst(s) employed in the hydrogenation unit is a palladium based catalyst.

7. Process according to claim 6 wherein the palladium based catalyst comprises palladium and preferably rhenium and/or silver supported on carbon.

8. Process according to any of the preceding claims wherein the hydrogenation unit is operated in a mixed gas/liquid phase.

9. Process according to any of the preceding claims wherein the feed mixture introduced into the reactor(s) is always below its dew point temperature.

10. Process according to any of the preceding claims wherein the molar ratio of hydrogen to [ethanoic acid and ethyl ethanoate] that is introduced into the hydrogenation unit, is greater than 2:1, preferably the molar ratio is greater than 4:1 1 and most preferably the molar ratio is greater thian 5:1; and is less than 100:1, preferably less than 50:1 and most preferably less than 15:1.

11. Process according to any of the preceding claims wherein the CO fed to the carbonylation reactor and the H2 fed to the hydrogenation unit emanates from the separation of the synthesis gas produced in step1 of claim 3.
